# EUROPÄISCHE PATENTSCHRIFT

(11) **EP 0 780 376 B1**
(45) Veröffentlichungstag und Bekanntmachung des Hinweises auf die Patenterteilung: **16.10.2002**
(21) Anmeldenummer: 96120138.1
(22) Anmeldetag: 14.12.1996
(51) Int. Cl.: C07D 219/08, C08K 5/3437, G03F 7/031

(54) **2-Acylamino-9-aryl-acridine, Verfahren zu ihrer Herstellung und diese enthaltende lichtempfindliche Gemische**
2-Acylamino-9-aryl-acridines, process for their preparation, and light-sensitive compositions containing them
2-Acylamino-9-aryl-acridines, procédé pour leur préparation et compositions sensibles à la lumière les contenant

(30) Priorität: 23.12.1995 DE 19548623
(43) Veröffentlichungstag der Anmeldung: 25.06.1997
(73) Patentinhaber: AGFA-GEVAERT, 2640 Mortsel (BE)
(72) Erfinder: Scheler, Siegfried, Dr., deceased (DE); Bergmann, Klaus-Peter, 55128 Mainz (DE); Buhr, Gerhard Dr., 61462 Königstein (DE)

(56) Entgegenhaltungen:
- EP-A- 0 374 704
- DE-A- 2 027 467
- DE-A- 2 328 195
- DE-A- 2 361 041
- DE-B- 1 283 091
- ACTA PHARMACEUTICA JUGOSLAVICA, Bd. 37, Nr. 3, 1987, Seiten 157-63, XP000197269 A.H. BEDAIR ET AL.: "Synthesis of some derivatives of 4-acetylamino-3-nitro- benzenesulfonic acid, part 2"
- JOURNAL OF THE CHEMICAL SOCIETY, PERKIN TRANSACTIONS I, 1978, Seiten 1211-8, XP002029114 R.N. CARDE ET AL.: "Intramolecular Nitrene Insertions into Aromatic and Heteroaromatic Systems. Part 4. Insertions using Triphenylmethanes, Unactivated or Bearing Electron-donating Groups"

## Beschreibung

Die Erfindung betrifft 2-Acylamino-9-aryl-acridine, ein Verfahren zu ihrer Herstellung und lichtempfindliche Gemische, die diese Verbindungen enthalten.

Aus der DE-C 20 27 467 sind photopolymerisierbare Gemische bekannt, die polymere Bindemittel, radikalisch polymerisierbare Verbindungen mit mindestens einer endständigen ethylenisch ungesättigten Gruppe und einem Siedepunkt oberhalb 100 °C bei Normaldruck und eine 9-Aryl-acridinverbindung als Photoinitiator enthalten. Von diesen Verbindungen zeichnet sich besonders das 9-Phenyl-acridin durch seine hohe Lichtempfindlichkeit aus. Diese Verbindung und andere bevorzugte Vertreter dieser Klasse haben den Nachteil, daß sie dazu neigen, aus photopolymerisierbaren Gemischen, die in Kontakt mit Polyethylenfolien stehen, in diese Folien und durch sie hindurch zu wandern bzw. zu diffundieren. Dadurch verarmt die Schicht an Initiator und verliert Empfindlichkeit.

Ein weiterer Nachteil der bekannten Gemische besteht darin, daß sie bei Anwendungen, für die sie im Verlaufe der Verarbeitung längere Zeit auf höhere Temperaturen erhitzt werden, z.B. bei der Herstellung von Lötstoppmasken (solder mask), durch Sublimation Photoinitiator verlieren. Das kann einerseits zu Lichtempfindlichkeitsverlust führen, wenn sich noch ein Photovernetzungsschritt anschließen soll; es führt aber auch zu Verschmutzung und bzw. oder Korrosion der Verarbeitungsgeräte und der damit herzustellenden elektronischen Bauteile.

In der EP-A 374 704 sind in 2-Stellung durch eine Alkyl- oder Acylgruppe substituierte 9-Aryl-acridine beschrieben, die sich als Photoinitiatoren in photopolymerisierbaren Gemischen eignen und die eine geringere Diffusionsneigung und eine geringere Neigung zum Ausbluten in sauren galvanischen Bädern als unsubstituierte 9-Aryl-acridine aufweisen. Eine geringere Sublimationsneigung ist für diese Verbindungen nicht beschrieben.

In *Acta Pharm. Jugosl*. **37** [1987] 157 - 163 sind biologisch aktive Derivate der 4-Acetylamino-3-nitro-benzolsulfonsäure offenbart, darunter 4-Acetylamino-3-nitro-N-(9-phenyl-acridin-2-yl)-benzolsulfonamid (dort ist eine Verbindung IIIb genannt, bezeichnet als "N¹-2'-(10'-phenylacridinyl)-3-nitro-4-acetylaminobenzene-sulfonamide").

Aufgabe der Erfindung war es, als Photoinitiatoren in photopolymerisierbaren Gemischen geeignete substituierte 9-Aryl-acridine bereitzustellen, die beim Einsatz in derartigen Gemischen, insbesondere für die Herstellung von Photoresists und Lötstoppmasken, nicht nur eine geringere Diffusionsneigung sondern auch eine geringere Neigung zur Sublimation aus der Schicht bei erhöhter Temperatur im unbelichteten und belichteten Zustand aufweisen.

Gemäß der Erfindung werden neue Verbindungen der allgemeinen Formel I 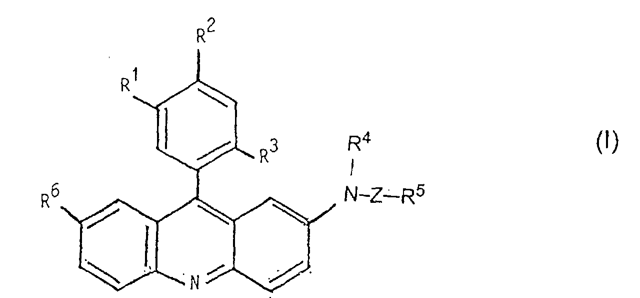 vorgeschlagen, worin
- R¹, R², R³ und R⁶: untereinander gleich oder verschieden sind und Wasserstoffoder Halogenatome, Alkyl-, Alkoxy- oder Alkoxyalkylgruppen mit 1 bis 6 Kohlenstoffatomen,
- R⁴: ein Wasserstoffatom, eine Alkylgruppe mit 1 bis 4 Kohlenstoffatomen oder einen Rest SO₂R⁵,
- R⁵: eine Alkyl-, Alkoxy-, Alkoxyalkyl-, Carboxyalkyl- oder Alkenylgruppe mit bis zu 6 Kohlenstoffatomen, eine Aralkylgruppe mit 7 bis 10 Kohlenstoffatomen oder eine Aralkenylgruppe mit 8 bis 10 Kohlenstoffatomen und
- Z: eine Carbonyl- oder Sulfonylgruppe
bedeuten.

Erfindungsgemäß wird auch ein photopolymerisierbares Gemisch vorgeschlagen, insbesondere ein photopolymerisierbares Gemisch, das ein polymeres Bindemittel und eine ethylenisch ungesättigte, radikalisch polymerisierbare Verbindung enthält und das durch einen Gehalt an einer Verbindung gemäß vorstehender Formel I als Photoinitiator gekennzeichnet ist.

Erfindungsgemäß wird schließlich auch ein Verfahren zur Herstellung einer Verbindung der allgemeinen Formel I vorgeschlagen, das darin besteht, daß man 2-Chlor-5-nitro-benzoesäure mit einem Chlorierungsmittel in das Säurechlorid umwandelt und dieses in Gegenwart eines Friedel-Crafts-Katalysators mit einer Verbindung der Formel II 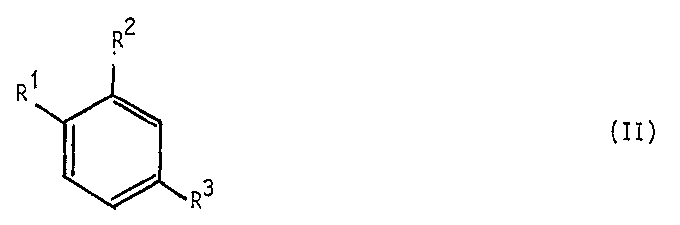 zum substituierten Benzophenon der Formel III 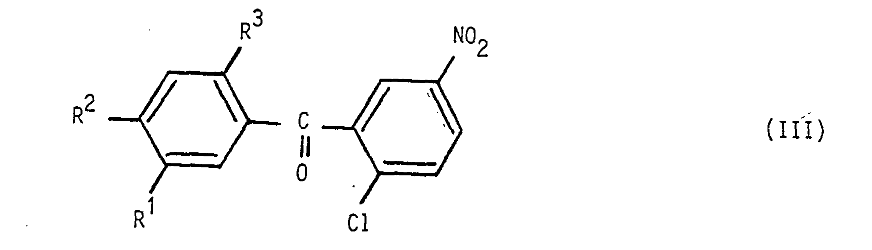 umsetzt, das 2-Chloratom der Verbindung III durch Umsetzen mit einem aromatischen Amin der Formel IV 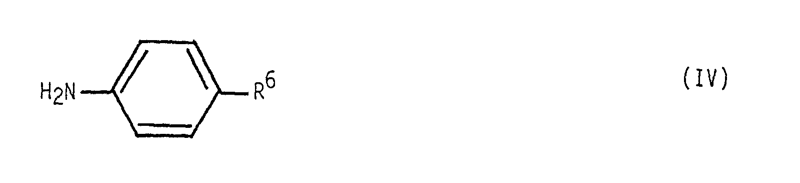 gegen einen Anilinorest austauscht, das erhaltene sekundäre aromatische Amin der Formel V 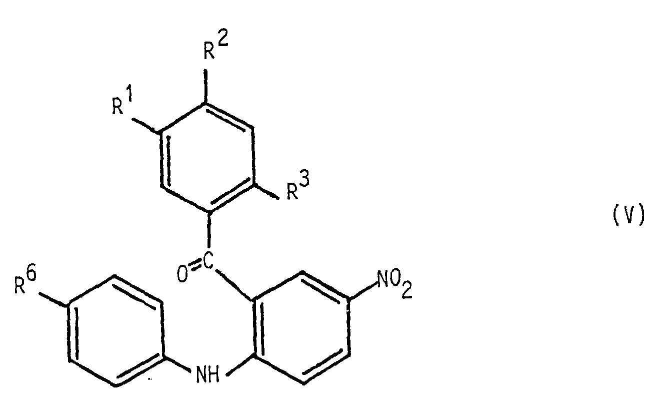 zum 2-Nitro-9-aryl-acridin der Formel VI 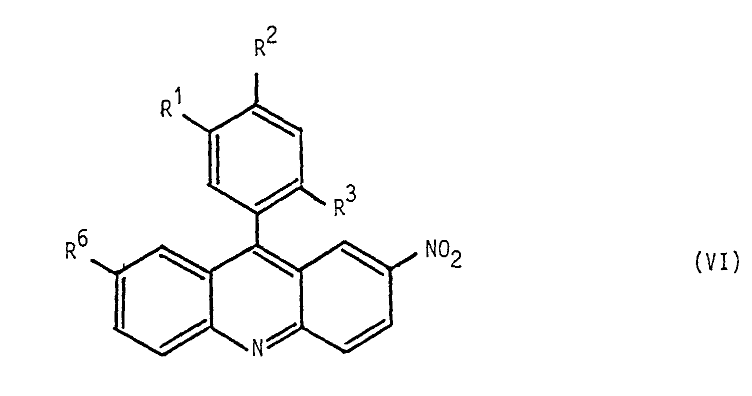 cyclisiert, die Nitroverbindung VI zum entsprechenden primären Amin (VII) reduziert und das Amin VII mit einem Acylierungsmittel R⁵ - Z - X und ggf. mit einem weiteren Acylierungsmittel oder einem Alkylierungsmittel R⁴ - X, worin X ein Halogenatom bedeutet, zur Verbindung der Formel I umsetzt.

Wenn in den erfindungsgemäßen Verbindungen R¹, R², R³ und/oder R⁶ Halogenatome sind, sind sie bevorzugt Chlor- oder Brom-, insbesondere Chloratome. Als Alkylgruppen werden solche mit 1 bis 4, insbesondere 1 oder 2 Kohlenstoffatomen, vor allem Methylgruppen bevorzugt. Auch als Alkoxy- oder Alkoxyalkylgruppen sind solche mit bis zu 4, besonders bis zu 2 Kohlenstoffatomen vorteilhaft. Von diesen Resten ist vorzugsweise mindestens einer ein Wasserstoffatom.
R⁴ ist bevorzugt Wasserstoff, kann aber auch eine niedere Alkylgruppe mit vorzugsweise 1 oder 2 Kohlenstoffatomen, insbesondere eine Methylgruppe, oder aber eine Gruppe R⁵SO₂, vorzugsweise eine Alkansulfonyl-, insbesondere Methansulfonylgruppe sein.
R⁵ kann eine Alkylgruppe mit 1 bis 6, vorzugsweise 1 bis 4, insbesondere 1 oder 2 Kohlenstoffatomen oder ein Alkoxy- oder Alkoxyalkylgruppe entsprechender Kettenlänge sein. Carboxyalkyl- oder Alkenylgruppen R⁵ können 2 bis 6, vorzugsweise 2 bis 4 Kohlenstoffatome enthalten. Als Aralkylgruppen werden Benzylgruppen, als Aralkenylgruppen Phenylethenylgruppen bevorzugt. Z ist vorzugsweise eine Carbonylgruppe.

Die Herstellung der erfindungsgemäßen Verbindungen erfolgt in einer Folge von einfach durchzuführenden Stufen mit hoher Ausbeute.
Zunächst wird die 2-Chlor-5-nitrobenzoesäure mit Phosphorpentachlorid oder vorzugsweise mit Thionylchlorid in Gegenwart von N-Methylmorpholin in das Carbonsäurechlorid übergeführt. Ohne Zwischenisolierung wird dieses mit der aromatischen Verbindung II unter Zusatz von wasserfreiem FeCl₃ zu dem entsprechend substituierten Benzophenon (III) umgesetzt. Aus dem mit einem polaren Lösemittel, z.B. Ethanol, verdünnten Reaktionsgemisch fällt das Reaktionsprodukt in sehr reiner kristalliner Form aus.

Die Verbindung III wird mit dem aromatischen Amin IV bei erhöhter Temperatur in Anwesenheit von Natriumacetat zum Diphenylaminderivat (V) umgesetzt. Auch dieses Produkt fällt in hoher Reinheit und guter Ausbeute aus dem verdünnten Reaktionsgemisch aus.
Die Verbindung V wird in saurem Reaktionsmedium, z.B. Eisessig/konz.Schwefelsäure, bei erhöhter Temperatur zum Acridinderivat (VI) cyclisiert.

Das 2-Nitro-9-phenyl-acridin wird dann zum primären Amin (VII) reduziert. Das kann in Ethanol/konz.Salzsäure mit SnCl₂ oder in stark saurer wäßriger Lösung mit metallischem Aluminium (Aluminiumgrieß) erfolgen. Die letztere Methode hat den Vorteil, daß keine umweltbelastenden Schwermetallsalze anfallen. Die Verbindung VII wird ebenfalls in hoher Ausbeute erhalten.
Schließlich wird das primäre Amin VII mit einem Carbonsäureanhydrid (z.B. Essigsäure-, Bernsteinsäure- oder Methacrylsäureanhydrid), einem Carbonsäurehalogenid, insbesondere -chlorid (z.B. Methoxyessigsäure-, Phenylessigsäure-, Zimtsäure- oder Benzoesäurechlorid), einem Carbonsäureesterchlorid (z.B. Chlorameisensäureethylester) oder mit einem Alkyl- oder Arylsulfonsäurechlorid ggf. in einem Lösemittel und ggf. in Gegenwart einer tertiären Base (Triethylamin, Pyridin, N-Methylmorpholin oder 1,4-Diaza-bicyclo[2.2.2]octan) zur Verbindung I acyliert. Verbindungen mit R⁴ = R⁵Z werden am einfachsten unter geeigneten Bedingungen mit einem Überschuß an Acylierungsmittel erhalten. Alkylreste R⁴ können auch vor der Acylierung mit einem Alkylierungsmittel eingeführt werden. Acylreste R⁴≠R⁵Z lassen sich in einem getrennten Acylierungschritt vor oder nach dem Rest R⁵Z erzeugen.

Die erfindungsgemäßen Verbindungen weisen eine hervorragende Aktivität als radikalbildende Photoinitiatoren auf. Ihre Aktivität entspricht im wesentlichen der des unsubstituierten 9-Phenylacridins, die in der DE-C 20 27 467 beschrieben ist. Zur Herstellung der erfindungsgemäßen photopolymerisierbaren Gemische werden die Initiatoren zusammen mit einem organischen polymeren Bindemittel, einer radikalisch polymerisierbaren ethylenisch ungesättigten Verbindung und ggf. weiteren üblichen Bestandteilen in einem geeigneten Lösemittel gelöst; die erhaltene Lösung wird auf einen Schichtträger aufgebracht und zu einer photopolymerisierbaren Schicht getrocknet. Der Mengenanteil der Verbindungen der Formel I in dem erfindungsgemäßen Gemisch beträgt im allgemeinen 0,01 bis 10, vorzugsweise 0,1 bis 5 Gew.-%, bezogen auf die nichtflüchtigen Bestandteile.

Für die Zwecke der Erfindung geeignete polymerisierbare Verbindungen sind bekannt und z.B. in den US-A 2 760 863 und 3 060 023 beschrieben.
Bevorzugte Beispiele sind Acryl- und Methacrylsäureester von ein- oder mehrwertigen, bevorzugt mindestens zweiwertigen Alkoholen, wie Ethylenglykoldiacrylat, Polyethylenglykoldimethacrylat, Acrylate und Methacrylate von Trimethylolethan, Trimethylolpropan, Pentaerythrit und Dipentaerythrit und von mehrwertigen alicyclischen Alkoholen, oder N-substituierte Acryl- und Methacrylsäureamide. Mit Vorteil werden auch Umsetzungsprodukte von Mono- oder Diisocyanaten mit Partialestern mehrwertiger Alkohole eingesetzt. Derartige Monomere sind in den DE-A 20 64 079, 23 61 041 und 28 22 190 beschrieben. Der Mengenanteil der Schicht an Monomeren beträgt im allgemeinen etwa 10 bis 80, vorzugsweise 20 bis 60 Gew.-%.
Das Gemisch enthält außerdem noch ein polymeres Bindemittel. Als Bindemittel kann eine Vielzahl organischer Polymerisate, die in dem Beschichtungslösemittel löslich sind, Einsatz finden. Als Beispiele seien genannt: Polyamide, Polyvinylester, Polyvinylacetale, Polyvi-nylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.
Ferner kommen als Bindemittel Naturstoffe oder umgewandelte Naturstoffe in Betracht, z.B. Gelatine und Celluloseether.
Mit besonderem Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrig-alkalischen Entwicklern entwickeln lassen. Derartige Bindemittel können z.B. die folgenden Gruppen enthalten: -COOH, -PO₃H₂, -SO₃H; -SO₂NH-, -SO₂-NH-SO₂- und -SO₂-NH-CO-.
Als Beispiele hierfür seien genannt: Maleinatharze, Polymerisate aus N(p-Tolylsulfonyl)-carbaminsäure-β-(methacryloyloxy)-ethylester und Mischpolymerisate dieser und ähnlicher Monomerer mit anderen Monomeren sowie Vinylacetat/-Crotonsäure- und Styrol/Maieinsäureanhydrid-Mischpolymerisate. Alkylmethacrylat/Methacrylsäure-Mischpolymerisate und Mischpolymerisate aus Methacrylsäure, höheren Alkylmethacrylaten und Methylmethacrylat und/oder Styrol, Acrylnitril u.a., wie sie in den DE-A 20 64 080 und 23 63 806 beschrieben sind, werden bevorzugt.
Die Menge des Bindemittels beträgt im allgemeinen 20 bis 90, vorzugsweise 40 bis 80 Gew.-% der Bestandteile der Schicht.
Die photopolymerisierbaren Gemische können je nach geplanter Anwendung und je nach den gewünschten Eigenschaften verschiedenartige Stoffe als Zusätze enthalten. Beispiele sind:
Inhibitoren zur Verhinderung der thermischen Polymerisation der Monomeren, Wasserstoffdonatoren,
die spektrale Lichtempfindlichkeit derartiger Stoffe modifizierende Stoffe, Farbstoffe,
gefärbte und ungefärbte Pigmente,
Farbbildner,
Indikatoren,
Weichmacher, z.B. Polyglykole oder Ester der p-Hydroxybenzoesäure.

Diese Bestandteile sind zweckmäßig so auszuwählen, daß sie in dem für den Initiierungsvorgang wichtigen aktinischen Strahlungsbereich möglichst wenig absorbieren.
Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.
Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z.B. Elektronenstrahlen, gehärtet werden, auf dem Dentalgebiet und insbesondere als lichtempfindliches Aufzeichnungsmaterial auf dem Reproduktionsgebiet.
Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt: Aufzeichnungsschichten für die photomechanische Herstellung von Druckformen für den Hochdruck, den Flachdruck, den Tiefdruck, den Siebdruck, von Reliefkopien, z.B. Herstellung von Texten in Blindenschrift, von Einzelkopien, Gerbbildern, Pigmentbildern usw.. Weiter sind die Gemische zur photomechanischen Herstellung von Ätzreservagen, z.B. für die Fertigung von Namensschildern, von gedruckten Schaltungen und für das Formteilätzen, anwendbar. Besondere Bedeutung haben die erfindungsgemäßen Gemische als Kopierschichten für die photomechanische Herstellung von Flachdruckformen und für die Photoresisttechnik.
Die gewerbliche Verwertung des Gemischs für die genannten Anwendungsgebiete kann in der Form einer flüssigen Lösung oder Dispersion, z.B. als Photoresistlösung, erfolgen, die vom Verbraucher selbst auf einen individuellen Träger, z.B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z.B. für die Herstellung von Druckformen, vorliegen. Ebenso ist es für die Herstellung von Trockenresist geeignet.

Es ist im allgemeinen günstig, die Gemische während der Lichtpolymerisation dem Einfluß des Luftsauerstoffs weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z.B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z.B. Wachse, Polyvinylalkohol, Polyphosphate, Zucker usw.
Als Schichtträger für mit dem erfindungsgemäßen Gemisch hergestellte Kopiermaterialien sind beispielsweise Aluminium, Stahl, Zink, Kupfer und Kunststoffolien, z.B. aus Polyethylenterephthalat oder Celluloseacetat, sowie Siebdruckträger, wie Perlongaze, geeignet.
Die Herstellung der lichtempfindlichen Materialien unter Verwendung des erfindungsgemäßen Gemischs erfolgt in bekannter Weise.
So kann man dieses in einem Lösemittel aufnehmen und die Lösung bzw. Dispersion durch Gießen, Sprühen, Tauchen, Antrag mit Walzen usw. auf den vorgesehenen Träger als Film antragen und anschließend trocknen. Dicke Schichten (z.B. von 250 µm und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und getrocknet. Die lichtempfindlichen Schichten - Dicke etwa zwischen 10 und 100µm - werden dann gleichfalls zunächst durch Laminieren zusammen mit dem temporären Träger auf das gewünschte Substrat laminiert.
Die Verarbeitung der Materialien wird in bekannter Weise vorgenommen. Zur Entwicklung werden sie mit einer geeigneten Entwicklerlösung, bevorzugt einer schwach alkalischen wäßrigen Lösung, behandelt, wobei die unbelichteten Anteile der Schicht entfernt werden und die belichteten Bereiche der Kopierschicht auf dem Träger zurückbleiben.
Die erfindungsgemäßen Aufzeichnungsmaterialien zeichnen sich durch einen
geringeren Lichtempfindlichkeitsverlust beim Lagern aus. Dieser Vorteil wird offenbar durch eine höhere Diffusionsfestigkeit der Initiatoren in der photopolymerisierbaren Schicht gegenüber dem unsubstituierten 9-Phenyl-acridin bewirkt. Sie haben den weiteren Vorteil, daß die darin enthaltenen Photoinitiatoren auch bei höheren Temperaturen bis zu etwa 200 °C und darüber, wie sie z.B. bei der Herstellung von Lötstoppmasken auftreten, nicht oder zumindest in wesentlich geringerem Maße als die bisher bekannten Initiatoren vergleichbarer Wirksamkeit aus der unbelichteten oder belichteten Schicht sublimieren.
Im folgenden werden Beispiele für das erfindungsgemäße Gemisch angegeben. Dabei wird zunächst die Herstellung von Verbindungen der Formel I beschrieben. Anschließend werden Beispiele für die Anwendung der neuen Verbindungen in photopolymerisierbaren Gemischen gegeben.
In den Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

### Herstellungsbeispiel 1

### Herstellung von 2-Acetylamino-9-(2,5-dimethyl-phenyl)-acridin (Verbindung 1).

a) 2-Chlor-5-nitro-2',5'-dimethyl-benzophenon (Verbindung 1a).
   203,5 Gt 2-Chlor-5-nitro-benzoesäure wurden unter Rühren in 144,2 Gt Thionylchlorid eingetragen und mit 0,5 Vt N-Methylmorpholin versetzt. Die Suspension wurde zum Sieden erhitzt und 3 1/2 Stunden unter Rückfluß gerührt. Während der Reaktion stieg die Siedetemperatur von 66 - 67 °C auf etwa 100 - 102 °C an und es entstand eine klare grüngelbe Lösung. Nach beendeter Umsetzung wurde auf 70 - 75 °C abgekühlt und das überschüssige Thionylchlorid im Vakuum entfernt. Anschließend wurden 116,6 Gt p-Xylol und 3 Gt wasserfreies FeCl₃ zugegeben, die gelbbraune Lösung wurde auf 100-102 °C erwärmt und 3 Stunden bei dieser Temperatur unter Chlorwasserstoffentwicklung gerührt.
   Nach Abschalten der Heizung wurden zu dem noch heißen Reaktionsgemisch schnell 500 Vt Ethanol gegeben und die Mischung im Verlauf von 4 Stunden auf 25 °C abkühlen gelassen. Das ausgefallene Produkt wurde abgesaugt, mit Ethanol, dann mit Wasser gewaschen und 24 Stunden bei 40 °C im Umluftschrank getrocknet.
   Ausbeute: 262 Gt (90,5 % der Th.) hellgelber Nadeln.
   Fp.: 89 - 91 °C.
b) 2-Anilino-5-nitro-2',5'-dimethyl-benzophenon (**Verbindung 1b**)
   144,7 Gt 2-Chlor-5-nitro-2',5'-dimethyl-benzophenon und 41,8 Gt wasserfreies Natriumacetat wurden in 140,2 Gt Anilin (99,5 %ig) suspendiert und 2 1/2 Stunden unter gutem Rühren auf 150 - 160 °C erwärmt. Das bei der Umsetzung gebildete Wasser wurde dabei abdestilliert. Die gelbbraune, trübe Lösung wurde auf 70 °C abgekühlt und mit 500 Vt Ethanol versetzt. Nach Stehen über Nacht wurde der gelbe Niederschlag abgesaugt, mit Ethanol, dann mit Wasser gewaschen und bei 50 °C im Umluftschrank getrocknet.
   Ausbeute: 164 Gt (95 % der Th.), gelbe Kristalle.
   Fp.: 127 - 129 °C.
c) 2-Nitro-9-(2,5-dimethyl-phenyl)-acridin (**Verbindung 1c**)
   173 Gt 2-Anilino-5-nitro-2',5'-dimethylbenzophenon wurden in 1 250 Gt Eisessig suspendiert. Zu der Suspension wurden 66 Vt Schwefelsäure (96 %ig) gegeben, und das Ganze wurde unter gutem Rühren 6 Stunden auf 118 - 119 °C erwärmt. Die gelbe Lösung wurde in 10 000 Vt Wasser getropft, der ausgefallene gelbe Niederschlag abgesaugt, gut mit Wasser gewaschen und 24 Stunden bei 100 °C im Vakuum getrocknet.
   Ausbeute: 161 Gt (98 % d.Th.), gelbes Pulver.
   Fp.: 171 - 173 °C.
d) 2-Amino-9-(2,5-dimethyl-phenyl)-acridin (**Verbindung 1d**)
   20 Gt SnCl₂ x 2 H₂O wurden in 50 Vt Ethanol bei 60 °C gelöst, und in die Lösung wurden 4,1 Gt 2-Nitro-9-(2,5-dimethyl-phenyl)-acridin eingerührt. Die Temperatur stieg dabei auf 75 - 76 °C an. In die tiefrote Lösung wurden 17 Vt Salzsäure (38 %ig) eingetropft und das Ganze 30 Minuten bei 72 - 75 °C nachgerührt. Anschließend wurden weitere 4,1 Gt des Acridinderivats zugesetzt und das Gemisch wurde nach Zutropfen von 26 Vt 38 %iger Salzsäure 10 Minuten bei 72 - 75 °C nachgerührt. Danach wurde 30 Minuten zum Sieden erhitzt, in 850 Vt Wasser und 85 Vt 25 %iger Ammoniaklösung eingerührt und mit Methylenchlorid ausgeschüttelt. Nach Trocknen der organischen Phase über wasserfreiem Natriumsulfat wurde das Lösemittel abdestilliert. Der rote Rückstand wurde mit Diethylether angerieben, abgesaugt und im Vakuum getrocknet.
   Ausbeute: 6,7 Gt (90 % d.Th.), oranges Pulver.
   Fp.: 162 - 164 °C.
e) 2-Acetylamino-9-(2,5-dimethyl-phenyl)-acridin **(Verbindung 1)**
   70 Gt 2-Amino-9-(2,5-dimethyl-phenyl)-acridin wurden in 70 Vt Essigsäureanhydrid gelöst und mit 0,5 Vt N-Methylmorpholin (99 %ig) versetzt. Die orange Lösung wurde 30 Minuten bei 27 °C, danach weitere 30 Minuten bei 50 °C gerührt und anschließend in 250 Vt Wasser eingegossen. Nach Zugabe von 150 Vt 25 %iger Ammoniaklösung wurde der Niederschlag abgesaugt und 24 Stunden bei 40 °C im Umluftschrank getrocknet.
   Ausbeute: 6,9 Gt (85,5 % d.Th.), gelbe Kristalle.
   Fp.: 236 - 238 °C.

### Herstellungsbeispiel 2

### Herstellung von 2-Benzoylamino-9-(2,5-dimethyl-phenyl)-acridin (Verbindung 2).

2,98 Gt 2-Amino-9-(2,5-dimethyl-phenyl)-acridin (Verbindung 1d) wurden in 75 Vt Methylenchlorid bei 23 °C gelöst und unter Rühren allmählich mit 2,1 Gt Benzoylchlorid versetzt. Die Temperatur stieg dabei auf 27 - 28 °C. Die Lösung wurde 4 Stunden gerührt, nach Stehen über Nacht mit Natronlauge geschüttelt und die organische Phase nach Trocknen über wasserfreiem Natriumsulfat abdestilliert.
Ausbeute: 3,96 Gt (98 % d.Th.), gelbgrüne Kristalle.
Fp.: 253 - 255 °C.

### Herstellungsbeispiel 3

### Herstellung von 2-Ethyloxycarbonylamino-9-(2,5-dimethyl-phenyl)-acridin (Verbindung 3).

1,42 Gt der Verbindung 1d und 2,78 Gt Chlorameisensäureethylester wurden in 100 Vt Acetonitril bei 25 °C gelöst und mit 4,85 Vt N-Methylmorpholin versetzt. Die Temperatur stieg dabei auf 38 °C. Die gelbe Lösung wurde 15 Minuten nachgerührt, dann in 400 Vt Wasser gegossen, das Gemisch 2 Stunden gerührt und der ausgefallene Rückstand abgesaugt.
Ausbeute: 1,3 Gt (72 % d.Th.).
Fp.: 200 - 202 °C.

### Herstellungsbeispiel 4

### Herstellung von 2-(N,N-Bis-methansulfonyl-amino)-9-(4-methyl-phenyl)-acridin (Verbindung 12).

5,68 Gt 2-Amino-9-(4-methyl-phenyl)-acridin (**Verbindung 12d**) wurden mit 20,3 Gt Methansulfonylchlorid und 22,4 Gt Diazabicyclo[2.2.2]octan in 120 Vt Acetonitril gelöst und die Lösung 2 Stunden am Rückfluß erhitzt. Nach Abkühlen auf 20 °C wurde filtriert und das rote Filtrat in 1 000 Vt 0,5 %iger Ammoniaklösung eingetropft. Nach Stehen über Nacht wurde der Niederschlag abgesaugt und aus 135 Vt Ethanol umkristallisiert.
Ausbeute: 1,76 Gt (20 % d.Th.), hellgelbe Nadeln.
Fp.: 217 - 219 °C.

### Herstellungsbeispiele 5 bis 20

Analog Beispiel 1 wurden Verbindungen der allgemeinen Formel 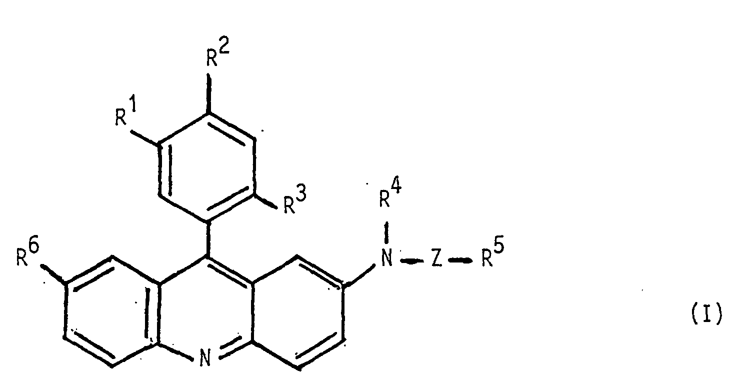 mit jeweils R⁴ = H hergestellt. In den folgenden Tabellen I bis V sind die Schmelzpunkte der Verbindungen 4 bis 11 und 13 bis 20 und ihrer Zwischenprodukte zusämmengestellt. Die Ausbeuteangaben beziehen sich auf die jeweilige Stufe. Die Bezeichnung der Zwischenprodukte entspricht in ihrer Ziffer dem oder den daraus hergestellten Endprodukt(en); der Buchstabe gibt die jeweilige Verfahrensstufe (a bis d) an.
Tabelle I - Zwischenprodukte der Stufe (a)
Tabelle II - Zwischenprodukte der Stufe (b)
Tabelle III - Zwischenprodukte der Stufe (c)
Tabelle IV - Zwischenprodukte der Stufe (d)
Tabelle V - Endprodukte - Stufe (e)

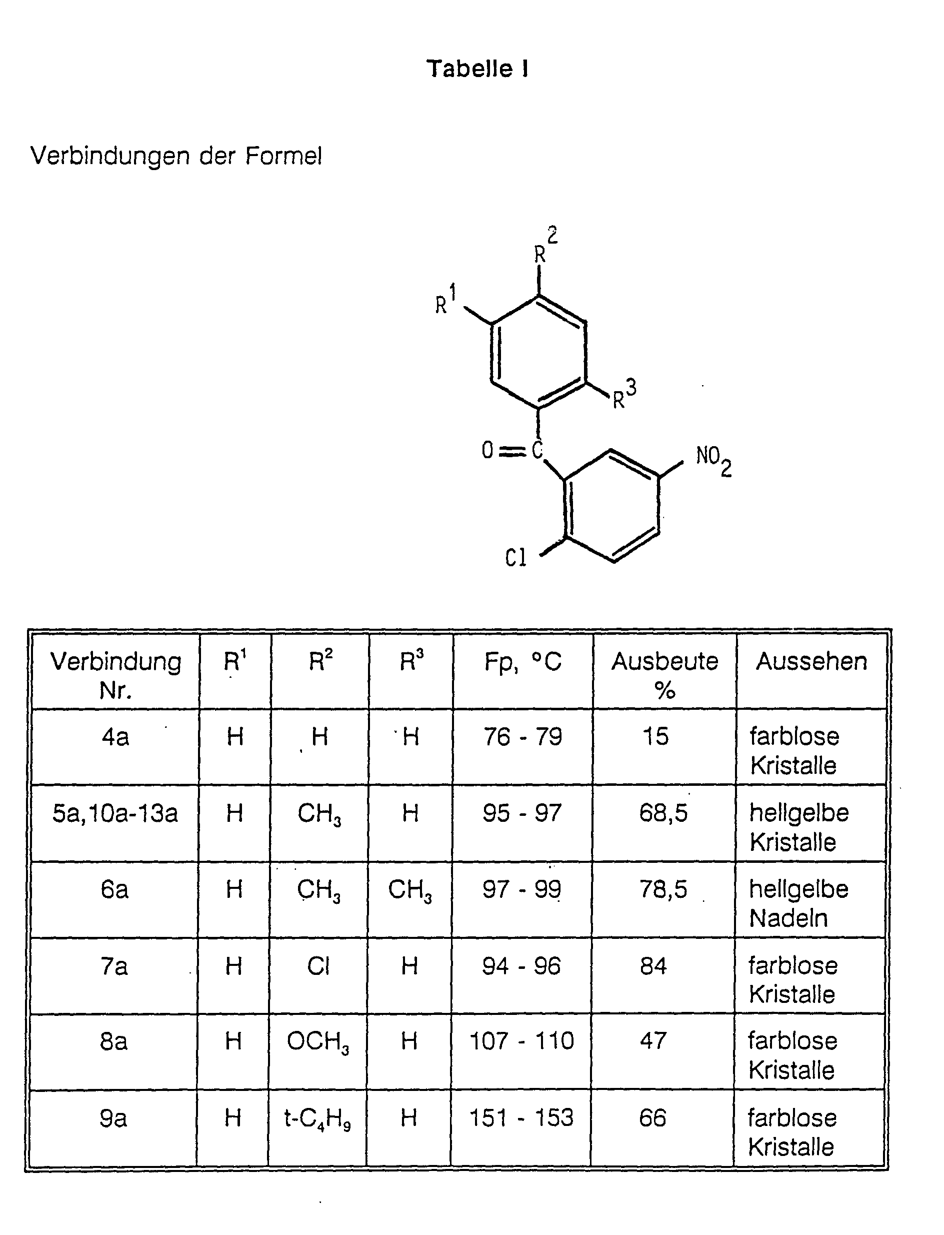

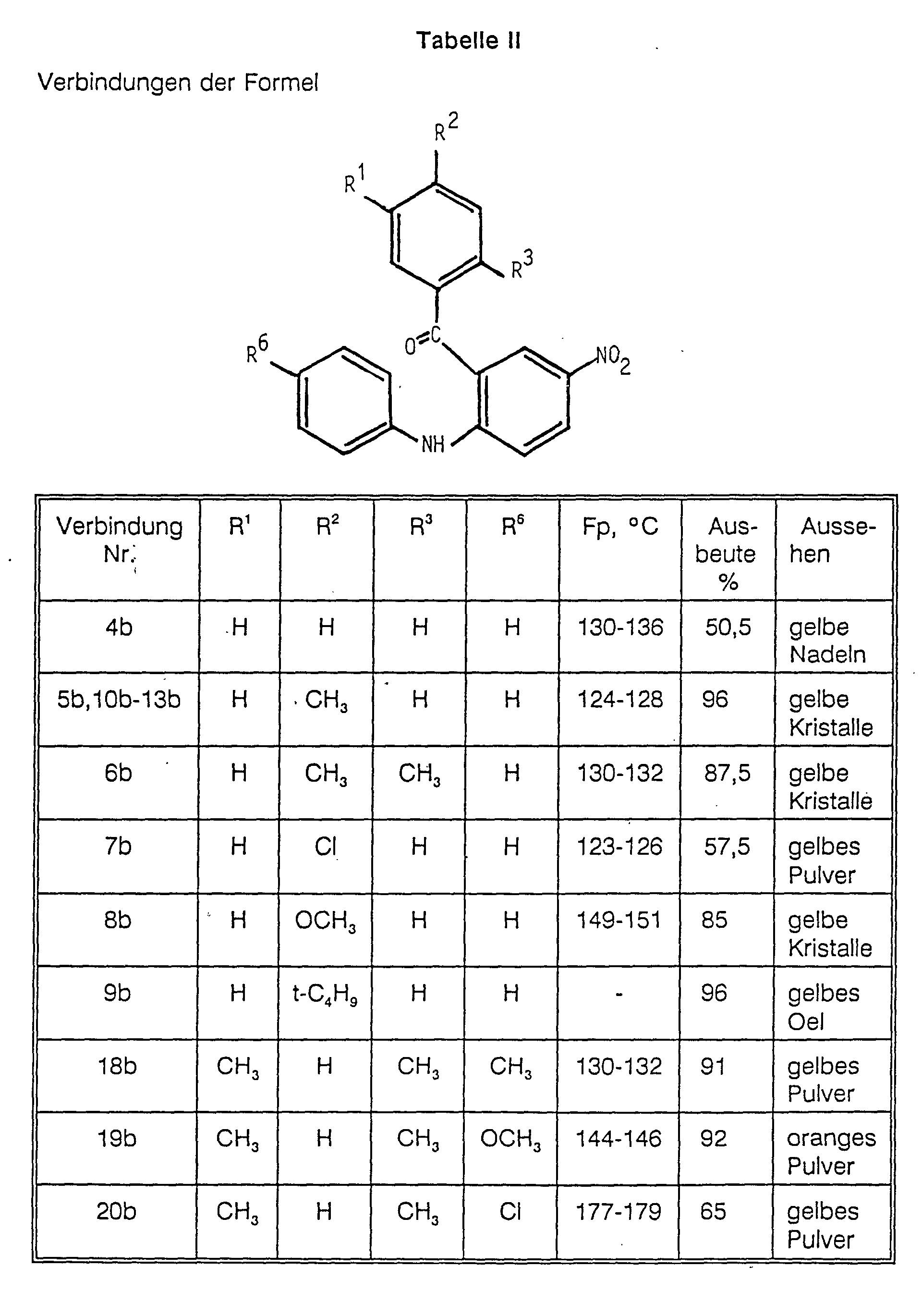

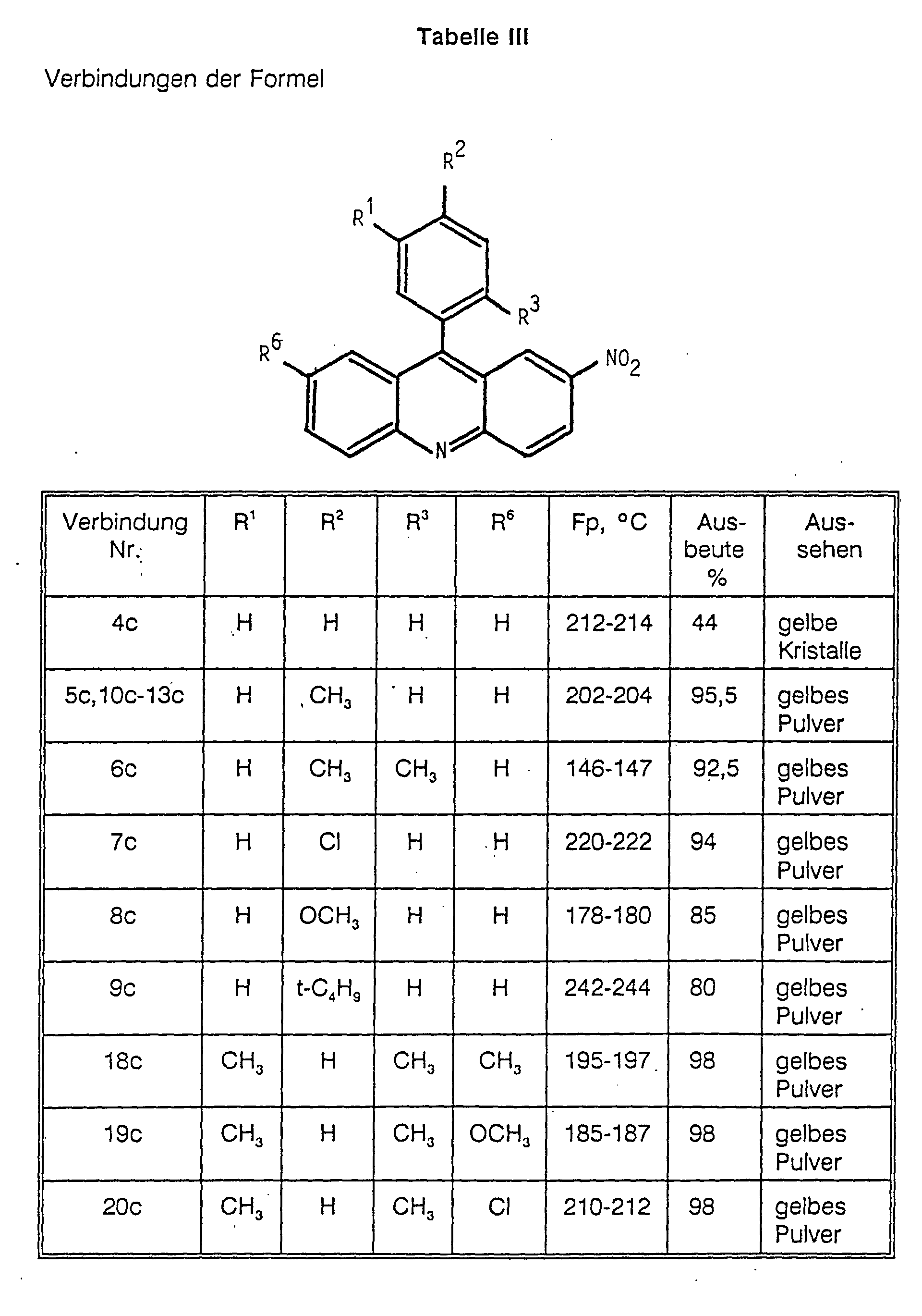

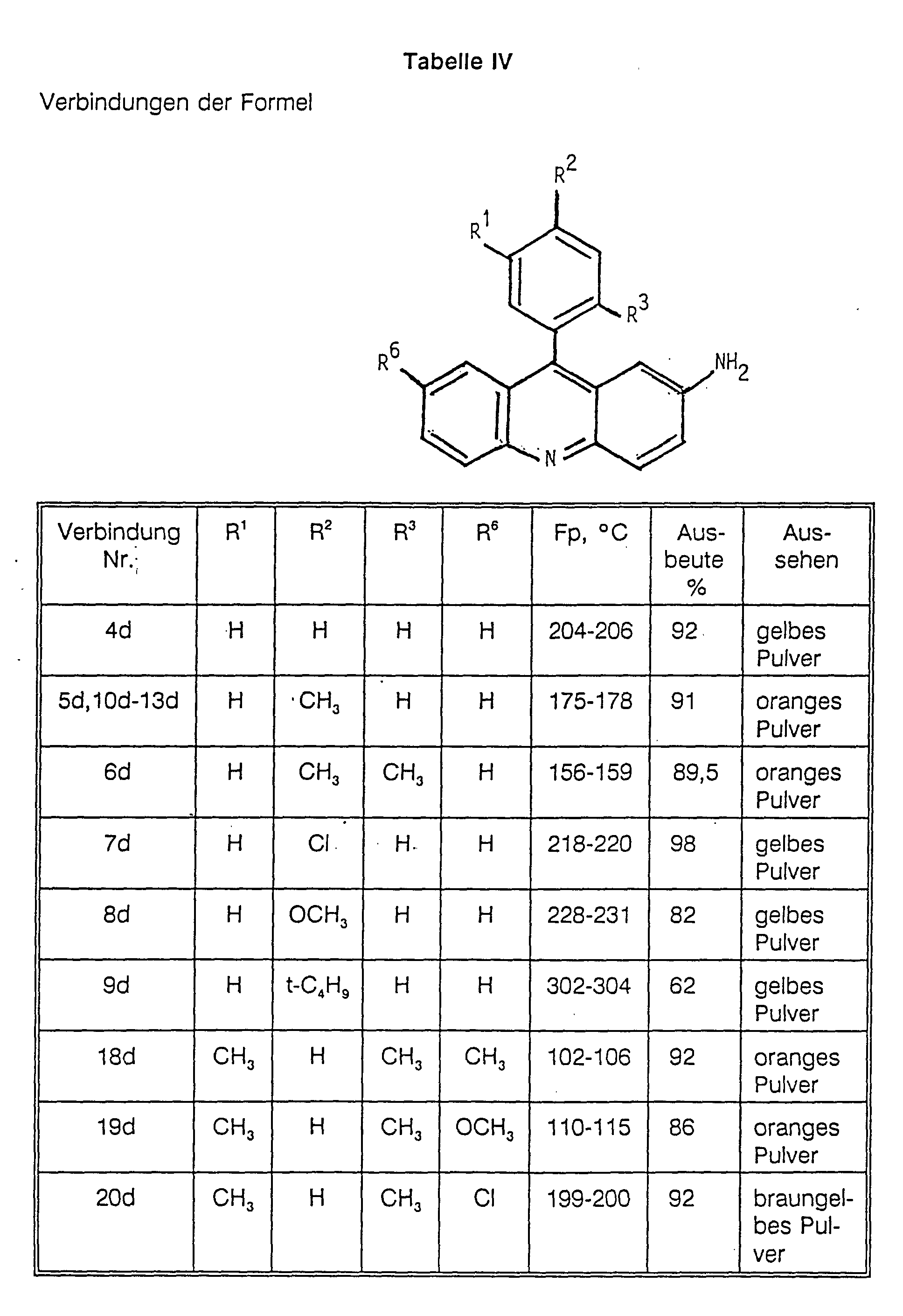

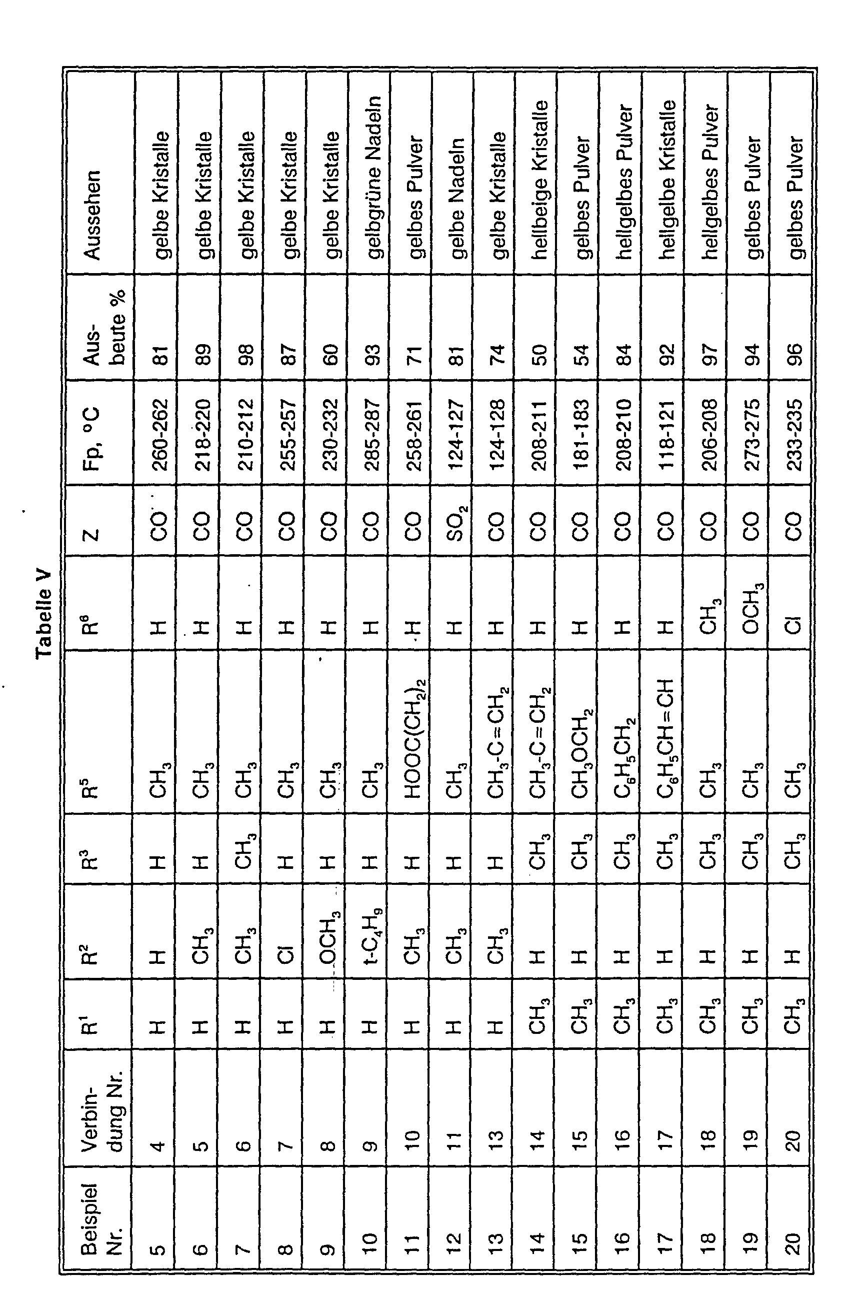

### Anwendungsbeispiele 1 - 9

Eine Grundlösung von

| | |
|---|---|
| 140,8 Gt | eines Polymeren aus Methacrylsäure, Methylmethacrylat, Hexylmethacrylat, Butylacrylat und Styrol (22 : 29 : 29 : 15 : 5), zugesetzt als 45 %ige Lösung (312,9 Gt) in Ethanol/Butanon 1:1, |
| 43,2 Gt | Trimethylolpropan-tris-acryloyloxyethylether, |
| 43,2 Gt | des Diurethans aus 2 mol Hydroxyethylacrylat und 1 mol 2,2,4-Trimethyl-hexamethylendiisocyanat, zugesetzt als 90 %ige Lösung (48 Gt) in Butanon, |
| 3,4 Gt | Leukokristallviolett, |
| 0,1 Gt | Victoriareinblau FGA (C.I. Basic Blue 81) und |
| 0,34 Gt | Tribrommethyl-phenylsulfon in |
| 300 Gt | Butanon |

wurde hergestellt. Zu je 75 Gt dieser Grundlösung wurden als Photoinitiatoren die in Tabelle VI aufgeführten Verbindungen in den dort angegebenen Mengen gegeben, die jeweils der gleichen molaren Menge entsprachen. Das Anwendungsbeispiel 1 (Verbindung PA) ist ein Vergleichsbeispiel.
Die Lösungen wurden auf biaxial verstreckte und thermofixierte Polyethylenterephthalatfolien von 25 µm Dicke so aufgeschleudert, daß nach dem Trocknen bei 100 °C jeweils ein Schichtgewicht von 45 g/m² erhalten wurde. Die Oberfläche der Schicht (A) wurde dann mit einer Polyethylenfolie kaschiert.
Zur Bestimmung der Lichtempfindlichkeit wurden die Photoresistschichten (A) nach Abziehen der Polyethylenfolien in einer handelsüblichen Laminier-Vorrichtung bei 115 °C auf mit 35 µm dicker Kupferfolie kaschierte Phenolharz-Schichtstoffplatten von 2 mm Dicke laminiert. Die Platten wurden mit einer 5 kW-Metallhalogenidlampe im Vakuumkopierrahmen 6 Sekunden belichtet. Als Vorlage diente ein 13-stufiger Belichtungskeil mit Dichtestufen von 0,15. Nach dem Entwickeln mit 1 %iger Sodalösung war in allen Fällen unter der Stufe 9 das Kupfer vollständig freigelegt.
Zur Prüfung der Diffusionsneigung wurde auf die Polyethylenfolie eines wie vorstehend hergestellten Sandwichs aus Polyesterfolie, Photoresistschicht A und Polyethylenfolie ein entsprechend hergestelltes lichtempfindliches Material aus Polyesterfolie und Photoresistschicht kalt laminiert, wobei die Photoresistschicht (B) diesmal aus der Grundlösung ohne Zusatz von Photoinitiator hergestellt worden war.
Das erhaltene Schichtgebilde wurde zwischen zwei Platten zusammengepreßt und 48 Stunden bei 40 °C gelagert. Danach wurden die beiden Photoresistschichten von der zentralen Polyethylenfolie getrennt und auf ihre UV-Absorption und Lichtempfindlichkeit untersucht. Hierbei wurde ermittelt, ob bei der Lagerung Photoinitiator aus der Photoresistschicht A durch die Polyethylenfolie in die initiatorfreie Photoresistschicht B diffundiert war. Es zeigte sich, daß nur aus der Vergleichsschicht des Beispiels 1 der Initiator in die initiatorfreie Testschicht B diffundiert war.
Auch die Bestimmung der UV-Absorption beider Schichten bei 360 nm zeigte das gleiche Verhalten.
Zur Bestimmung der Sublimationsfähigkeit der Photoinitiatoren wurde wie vorstehend ein Schichtgebilde mit einer Initiator enthaltenden Schicht (A) und einer initiatorfreien Photoresistschicht (B) hergestellt, wobei aber statt der zentralen Polyethylenfolie eine 3 mm dicke Polytetrafluorethylenplatte eingesetzt wurde, die mehrere rechteckige Öffnungen von 25 x 40 mm Größe aufwies und die an den Rändern der Probe mit Streifen aus doppelseitigem Klebeband gegen Verrutschen gesichert war. Das gesamte Schichtgebilde wurde 1 Stunde im Trockenschrank bei 150 °C gelagert. Dann wurden die photopolymerisierbaren Schichten auf ihre Lichtempfindlichkeit und UV-Absorption im Bereich der Öffnungen der Polytetrafluorethylenplatte untersucht. Die Ergebnisse zeigt Tabelle VI.

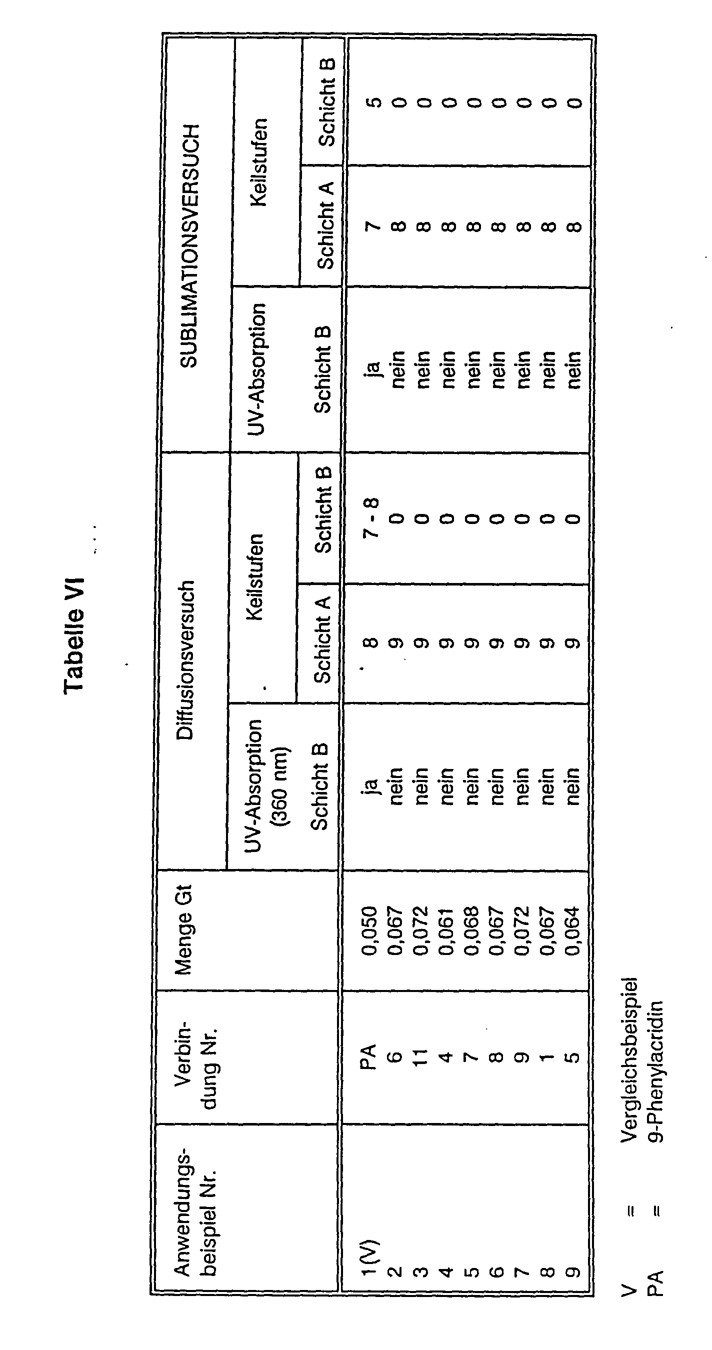

### Anwendungsbeispiele 10 - 18

Die in den Anwendungsbeispielen 1 bis 9 beschriebene Versuchsdurchführung wurde mit photopolymerisierbaren Materialien wiederholt, zu deren Herstellung die folgende Beschichtungslösung eingesetzt wurde:

| | |
|---|---|
| 13,4 Gt | eines Polymeren aus Methacrylsäure, Acrylsäure, Methylmethacrylat, Butylacrylat und Styrol (16 : 5 : 42 : 27 : 10), zugesetzt als 50 %ige Lösung (26,8 Gt) in Ethanol/Butanon 1 : 1, |
| 2,8 Gt | Trimethylolpropan-tris-acryloyloxyethylether, |
| 2,8 Gt | des Diurethans aus 2 mol Hydroxyethylmethacrylat und 1 mol 2,2,4-Trimethyl-hexamethylendiisocyanat, |
| 1,4 Gt | des Umsetzungsproduktes aus 1 mol Hydroxyethylmethacrylat, 5 mol Ethylenoxid und 1 mol Butylisocyanat, |
| y Gt | einer der in Tabelle VII angegebenen Verbindungen, |
| 0,008 Gt | Viktoriareinblau FGA, |
| 0,3 Gt | Leukokristallviolett und |
| 0,02 Gt | Tribrommethyl-phenylsulfon in |
| 15 Gt | Ethanol und |
| 15 Gt | Butanon. |

**Tabelle VII**

| Anwendungsbeispiel Nr. | Verbindung Nr. | Menge y (Gt) |
|---|---|---|
| 10 (V) | PA | 0,040 |
| 11 | 6 | 0,053 |
| 12 | 11 | 0,057 |
| 13 | 4 | 0,049 |
| 14 | 7 | 0,054 |
| 15 | 8 | 0,054 |
| 16 | 9 | 0,058 |
| 17 | 1 | 0,053 |
| 18 | 5 | 0,051 |

Nach 14 Sekunden Belichtung war in allen Fällen in Stufe 9 das Kupfer vollständig freigelegt. Nur im Vergleichsbeispiel 10 wurde eine Migration und Sublimation des Photoinitiators in die Schicht B beobachtet.

## Patentansprüche

1. Verbindungen der allgemeinen Formel 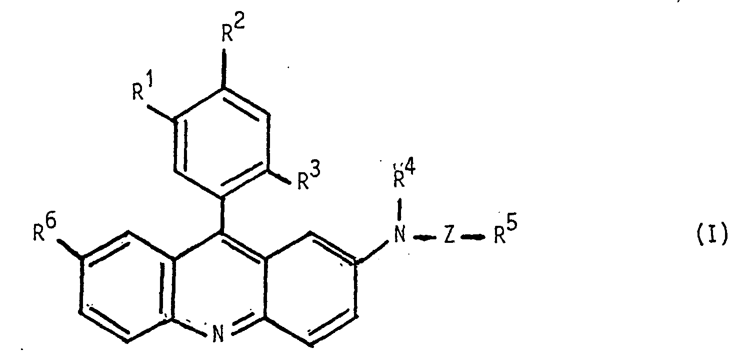 worin
R¹, R², R³ und R⁶ untereinander gleich oder verschieden sind und Wasserstoff- oder Halogenatome, Alkyl-, Alkoxy- oder Alkoxyalkylgruppen mit 1 bis 6 Kohlenstoffatomen,
R⁴ ein Wasserstoffatom, eine Alkylgruppe mit 1 bis 4 Kohlenstoffatomen oder einen Rest SO₂R⁵,
R⁵ eine Alkyl-, Alkoxy-, Alkoxyalkyl-, Carboxyalkyl- oder Alkenylgruppe mit bis zu 6 Kohlenstoffatomen, eine Aralkylgruppe mit 7 bis 10 Kohlenstoffatomen oder eine Aralkenylgruppe mit 8 bis 10 Kohlenstoffatomen und
Z eine Carbonyl- oder Sulfonylgruppe
bedeuten.

2. Verbindungen nach Anspruch 1, **dadurch gekennzeichnet, daß** R² ein Wasserstoffatom ist und R¹ und R³ Methylgruppen sind.

3. Verbindungen nach Anspruch 1, **dadurch gekennzeichnet, daß** R⁴ ein Wasserstoffatom und Z eine Carbonylgruppe ist.

4. Verbindungen nach einem der Ansprüche 1, 2 und 3, **dadurch gekennzeichnet, daß** R⁵ eine Methylgruppe ist.

5. Photopolymerisierbares Gemisch, **dadurch gekennzeichnet, daß** es eine Verbindung gemäß einem der Ansprüche 1 bis 4 als Photoinitiator enthält.

6. Photopolymerisierbares Gemisch nach Anspruch 5, **dadurch gekennzeichnet, daß** es ein polymeres Bindemittel und eine ethylenisch ungesättigte, radikalisch polymerisierbare Verbindung enthält.

7. Photopolymerisierbares Gemisch nach Anspruch 6, **dadurch gekennzeichnet, daß** die radikalisch polymerisierbare Verbindung ein Acryl- oder Methacrylsäureester eines mehrwertigen aliphatischen Alkohols ist.

8. Photopolymerisierbares Gemisch nach Anspruch 6, **dadurch gekennzeichnet, daß** das polymere Bindemittel wasserunlöslich und in wäßrig-alkalischen Lösungen löslich ist.

9. Verfahren zur Herstellung einer Verbindung der allgemeinen Formel I gemäß Anspruch 1, **dadurch gekennzeichnet, daß** man 2-Chlor-5-nitrobenzoesäure mit einem Chlorierungsmittel in das Säurechlorid umwandelt und dieses in Gegenwart eines Friedel-Crafts-Katalysators mit einer Verbindung der Formel II 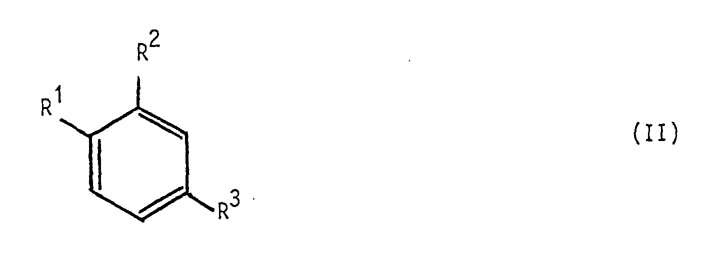 zum substituierten Benzophenon der Formel III 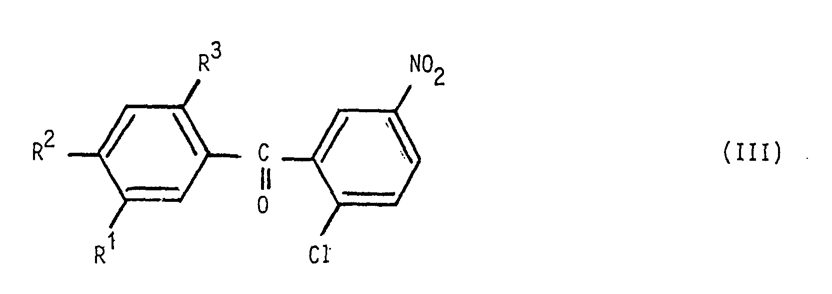 umsetzt, das 2-Chloratom der Verbindung III durch Umsetzen mit einem aromatischen Amin der Formel IV 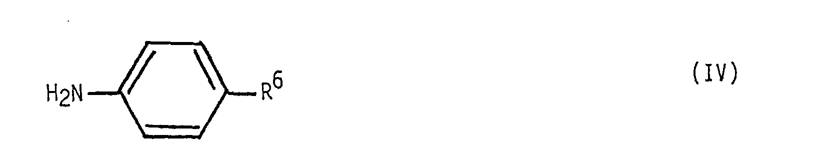 gegen einen Anilinorest austauscht, das erhaltene sekundäre aromatische Amin der Formel V 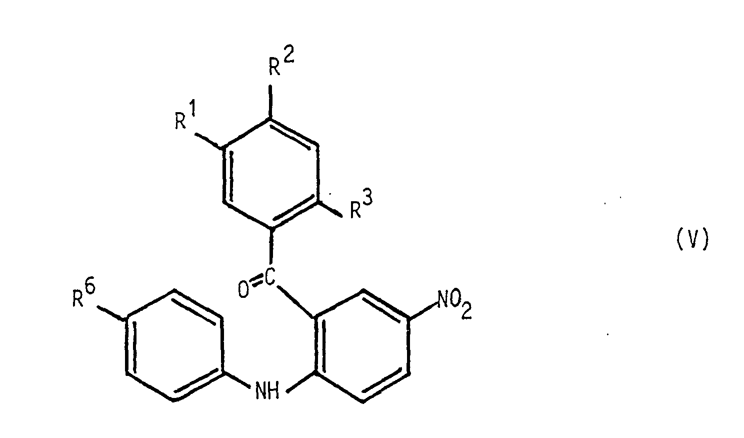 zum 2-Nitro-9-aryl-acridin der Formel VI 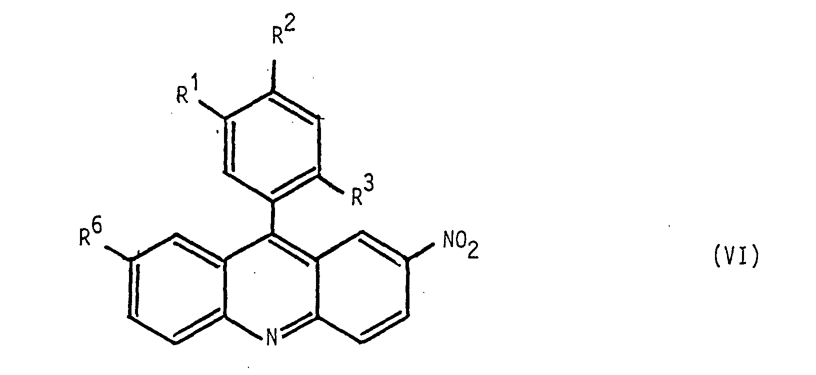 cyclisiert, die Nitroverbindung VI zum entsprechenden primären Amin (VII) reduziert und das Amin VII mit einem Acylierungsmittel R⁵ - Z - X und ggf. mit einem weiteren Acylierungsmittel oder einem Alkylierungsmittel R⁴ - X, worin X ein Halogenatom bedeutet, zur Verbindung der Formel I umsetzt.

## Claims

1. Compounds of the general formula 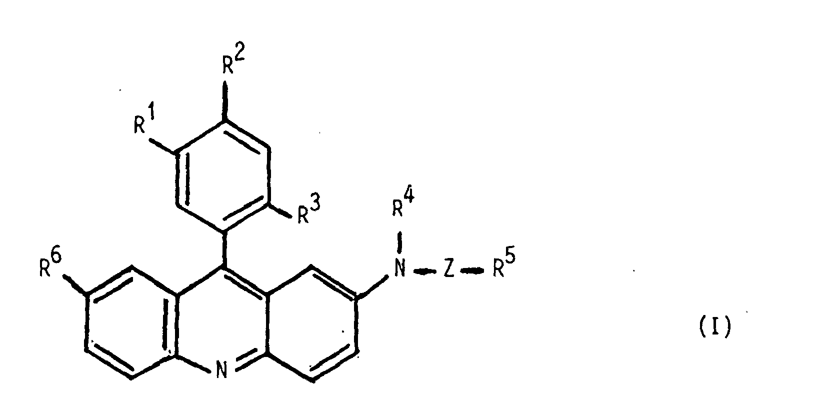 wherein
each of R¹, R², R³ and R⁶ same or different is selected from the group consisting of a hydrogen atom, a halogen atom, a C₁-C₆ alkyl group, a C₁-C₆ alkoxy group, and a C₁-C₆ alkoxyalkyl group;
R⁴ represents a hydrogen atom, a C₁-C₄ alkyl group or an SO₂R⁵ radical;
R⁵ represents an alkyl, alkoxy, alkoxyalkyl, carboxyalkyl or alkenyl group containing up to 6 carbon atoms, a C₇-C₁₀ aralkyl group or a C₈-C₁₀ aralkenyl group; and
Z represents a carbonyl or sulphonyl group.

2. Compounds according to claim 1, wherein R² is a hydrogen atom and R¹ and R³ are methyl groups.

3. Compounds according to claim 1, wherein R⁴ is a hydrogen atom and Z is a carbonyl group.

4. Compounds according to any of claims 1, 2 or 3, wherein R⁵ is a methyl group.

5. A photopolymerisable mixture, wherein said mixture comprises as photoinitiator a compound as defined in any of claims 1 to 4.

6. A photopolymerisable mixture according to claim 5, wherein said mixture further comprises a polymeric binder and an ethylenically unsaturated, free-radical-polymerisable compound.

7. A photopolymerisable mixture according to claim 6, wherein said free-radical-polymerisable compound is an acrylic ester or methacrylic ester of a polyhydric aliphatic alcohol.

8. A photopolymerisable mixture according to claim 6, wherein said polymeric binder is insoluble in water, and soluble in aqueous alkaline solutions.

9. A process for preparing a compound of general formula I as defined in claim 1, wherein said process comprises the steps of converting 2-chloro-5-nitrobenzoic acid to the acidic chloride using a chlorinating agent;
reacting the acidic chloride with a compound of the formula II 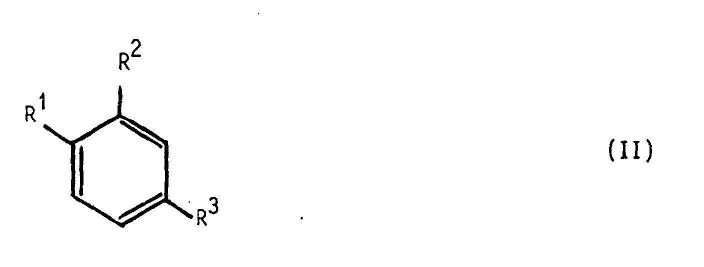 in the presence of a Friedel-Crafts catalyst to form the substituted benzophenone of the formula III; 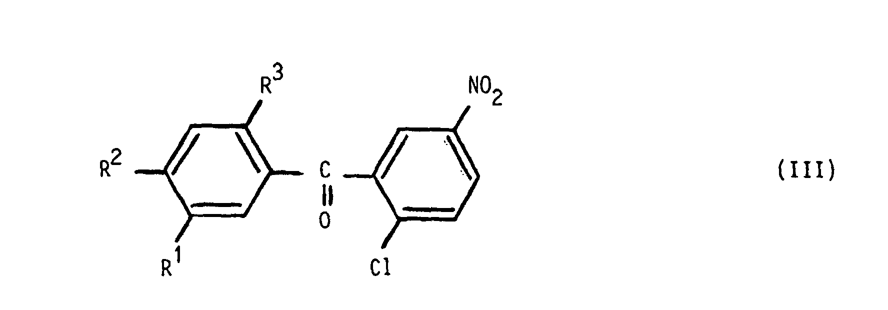 replacing the 2-chlorine atom of the compound III by an anilino radical as a result of reaction with an aromatic amine of formula IV 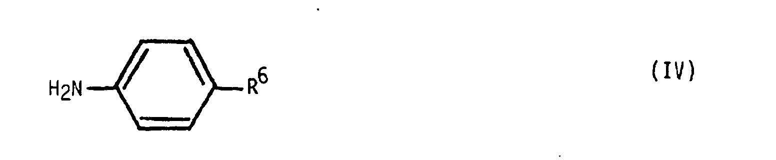 cyclising the secondary aromatic amine of the formula V obtained 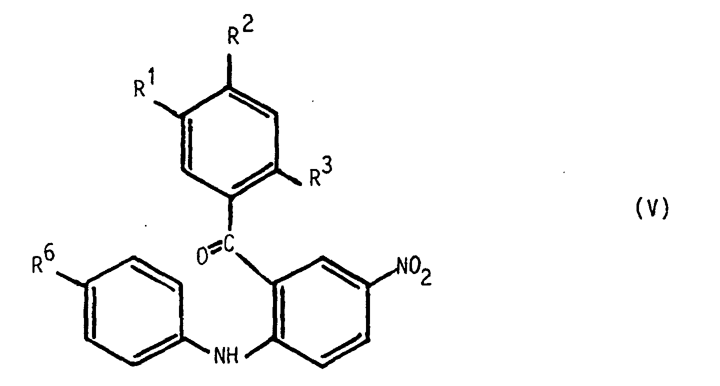 to form 2-nitro-9-arylacridine of the formula VI; 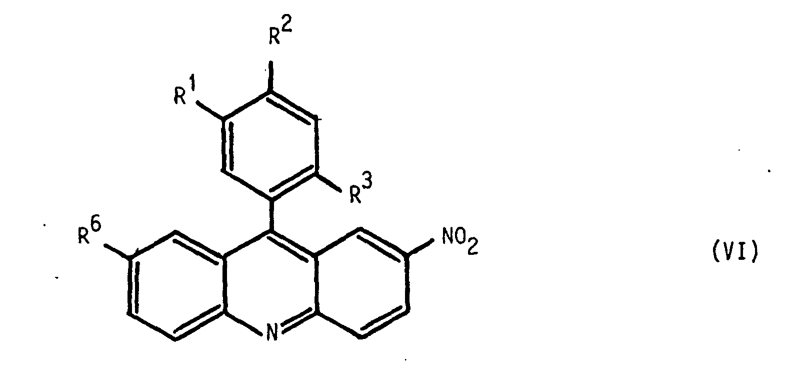 reducing the nitro compound VI to the corresponding primary amine (VII) and reacting the amine VII with an acylating agent R⁵-Z-X, and optionally with a further acylating agent or an alkylating agent R⁴-X, wherein X represents a halogen atom, to form the compound of the formula I.

## Revendications

1. Composés répondant à la formule générale 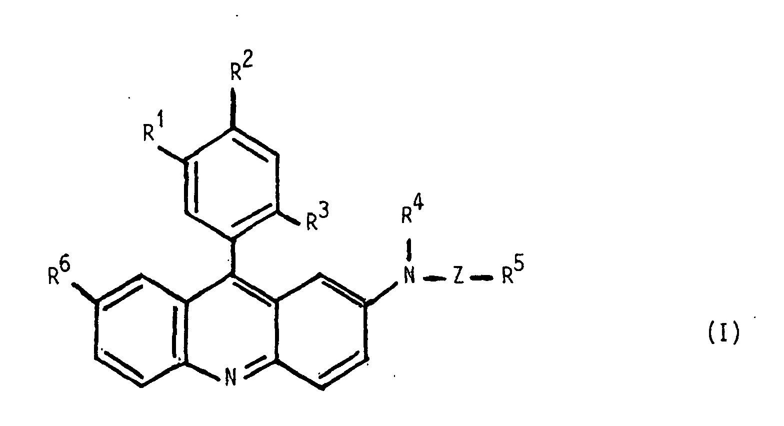 dans laquelle
R¹, R², R³ et R⁶ identiques ou différents sont choisis chacun parmi un atome d'hydrogène, un atome d'halogène, un groupe alkyle en C₁-C₆, un groupe alcoxy en C₁-C₆ et un groupe alcoxyalkyle en C₁-C₆;
R⁴ représente un atome d'hydrogène, un groupe alkyle en C₁-C₄ ou un radical SO₂R⁵;
R⁵ représente un groupe alkyle, alcoxy, alcoxyalkyle, carboxyalkyle ou alcényle contenant 6 atomes de carbone, un groupe aralkyle en C₇-C₁₀ ou un groupe aralcényle en C₈-C₁₀; et
Z représente un groupe carbonyle ou sulfonyle.

2. Composés selon la revendication 1, **caractérisés en ce que** R² est un atome d'hydrogène et R¹ et R³ sont des groupes méthyle.

3. Composés selon la revendication 1, **caractérisés en ce que** R⁴ est un atome d'hydrogène et Z est un groupe carbonyle.

4. Composés selon l'une quelconque des revendications 1, 2 ou 3, **caractérisés en ce que** R⁵ est un groupe méthyle.

5. Mélange photopolymérisable, **caractérisé en ce que** ledit mélange comprend en tant que photoamorceur un composé tel que défini dans l'une quelconque des revendications 1 à 4.

6. Mélange photopolymérisable selon la revendication 5, **caractérisé en ce que** ledit mélange comprend en outre un liant polymère et un composé polymérisable par voie radicalaire à insaturation éthylénique.

7. Mélange photopolymérisable selon la revendication 6, **caractérisé en ce que** ledit composé polymérisable par voie radicalaire est un ester acrylique ou un ester méthacrylique d'un alcool aliphatique polyhydroxylé.

8. Mélange photopolymérisable selon la revendication 6, **caractérisé en ce que** ledit liant polymère est insoluble dans l'eau et soluble dans les solutions alcalines aqueuses.

9. Procédé de préparation pour composés répondant à la formule générale I tels que définis dans la revendication 1, **caractérisé en ce que** ledit procédé comprend les étapes consistant à transformer l'acide 2-chloro-5-nitrobenzoïque en chlorure acide à l'aide d'un agent de chloruration;
faire réagir le chlorure acide avec un composé répondant à la formule II 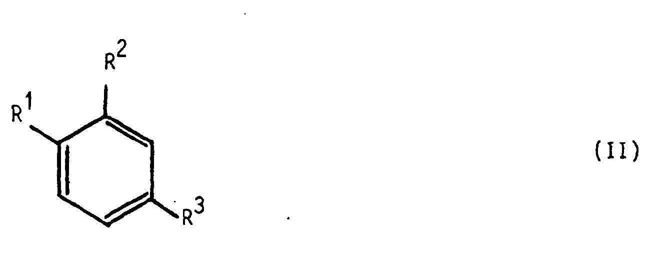 en présence d'un catalyseur Friedel-Crafts pour former la benzophénone substituée répondant à la formule III; 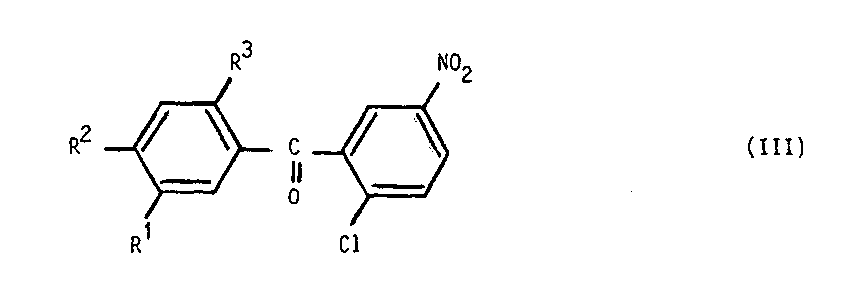 remplacer l'atome de 2-chlore du composé III par un radical aniline par réaction avec une amine aromatique répondant à la formule IV 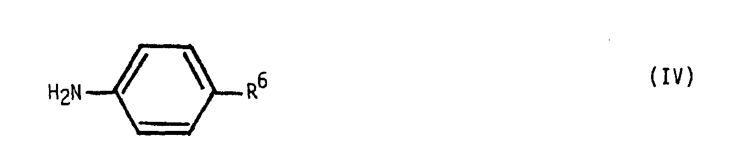 cycliser l'amine aromatique secondaire obtenue répondant à la formule V 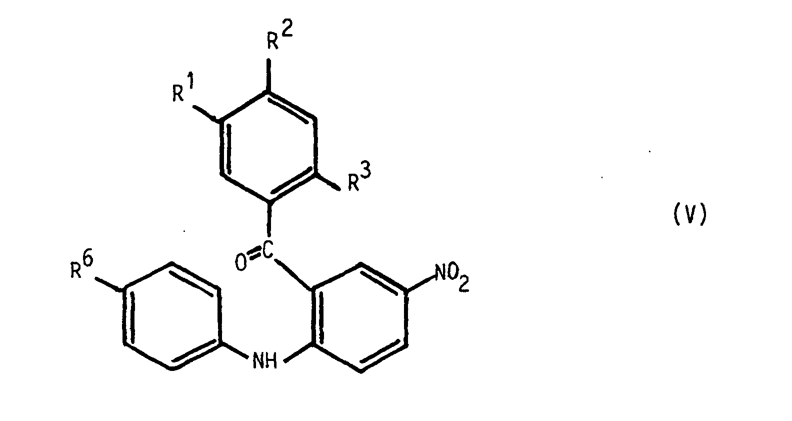 pour former la 2-nitro-9-arylacridine répondant à la formule VI; 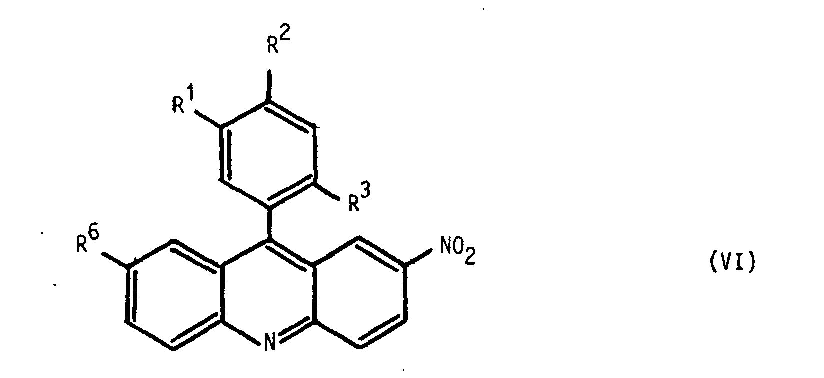 réduire le composé nitré VI à l'amine primaire correspondante (VII) et faire réagir l'amine VII avec un agent d'acylation R⁵-Z-X, et éventuellement avec un agent d'acylation additionnel ou un agent d'alkylation R⁴-X, dans lesquels X représente un atome d'halogène, pour former le composé répondant à la formule I.
